# EUROPÄISCHE PATENTSCHRIFT

(11) **EP 1 909 808 B1**
(45) Veröffentlichungstag und Bekanntmachung des Hinweises auf die Patenterteilung: **13.10.2010**
(21) Anmeldenummer: 06776627.9
(22) Anmeldetag: 04.08.2006
(51) Int. Cl.: A61K 36/73

(54) **VERFAHREN ZUR EXTRAKTION VON FRUCHTWACHSEN**
METHOD FOR OBTAINING FRUIT WAX
PROCEDE D'EXTRACTION DE CIRES DE FRUIT

(30) Priorität: 06.08.2005 DE 102005037210
(43) Veröffentlichungstag der Anmeldung: 16.04.2008
(73) Patentinhaber: Evonik Degussa GmbH, 45128 Essen (DE)
(72) Erfinder: WIESMÜLLER, Johann, 84518 Garching (DE); PILZ, Stephan, 70736 Fellbach (DE)
(86) Internationale Anmeldenummer: PCT/EP2006/007756
(87) Internationale Veröffentlichungsnummer: WO 2007/017205

(56) Entgegenhaltungen:
- WO-A-96/11043
- DE-A1- 3 101 025
- DE-A1- 4 206 154
- GB-A- 2 091 292
- US-A- 3 939 281
- US-A- 5 587 174

## Beschreibung

Gegenstand der vorliegenden Erfindung ist ein Verfahren zur Gewinnung von Fruchtwachsen aus pflanzlichen Bestandteilen durch Extraktion mit Hilfe von verdichtetem Propan und/oder Butan.

Fruchtwachse, insbesondere auf überwiegend natürlicher Basis, stellen zunehmend wichtige Ausgangsmaterialien zur Gewinnung von Wirkstoffen dar, die vor allem als Schutz- und Pflegewirkstoffe von der kosmetischen Industrie eingesetzt werden. Der Vorteil derartiger Komponenten ist insbesondere darin zu sehen, dass es sich dabei um nachwachsende Rohstoffe handelt, was sie unter ökologischen und ökonomischen Gesichtspunkten als äußerst wertvoll erscheinen lässt. Zudem steigt durch die Anwendung natürlicher Inhaltsstoffe die Verbraucherakzeptanz deutlich an, da die Verbraucherschaft zwischenzeitlich ein ausgeprägtes ökologisches Bewusstsein besitzt und Produkte für die Körper- und Gesundheitspflege äußerst kritisch auswählt.

Speziell im Bereich der Wachse und Paraffine werden Synonym unterschiedliche Begriffe eingesetzt, wobei man nach allgemein anerkannter Definition "Wachse" mit lipophilen Substanzen von plastischer Konsistenz, also wachsartiger Beschaffenheit, in Beziehung bringt. Bei Wachsen handelt es sich um Fettsäureester langkettiger oder cyclischer ein- oder zweiwertiger Alkohole. Diese Wachse sind meist von pflanzlicher oder tierischer Herkunft, womit sie typische Naturprodukte darstellen. Eine strenge Abgrenzung der pflanzlichen und tierischen Wachse aufgrund der an ihrem Aufbau beteiligten Fettsäuren und Fettalkoholen kann nicht vorgenommen werden. Es ist allerdings unstrittig, dass es sich bei Montansäure, Palmitinsäure und Stearinsäure um typische an diesem Naturwachsen beteiligte Fettsäuren handelt. Auf der Seite der Alkohole sind hier vor allem Cetylalkohol und Cerylalkohol zu nennen.

Echte Naturwachse pflanzlicher Herkunft sind bspw. Palmblätterwachse, wie Carnaubawachs, Palmwachs, Raffiawachs, Ouricourywachs, Gräserwachse, wie z. B. Candellilawachs, Espartowachs, Fiberwachs und Zuckerrohrwachs; Beeren- und Fruchtwachse sind bspw. Japanwachs, Bayberry-Wachs und Myrthenwachs. Das bekannteste Beispiel eines echten Naturwachses tierischer Herkunft ist das Bienenwachs, welches zur Hauptsache aus Palmitinsäure-Miricylester, also einer mit Miricylalkohol veresterten Palmitinsäure, besteht. Bekannt sind auch chinesische Insektenwachse, Schellackwachs und Wollwachse, wie sie bspw. aus Schafwolle gewonnen werden können.

Fruchtwachse und insbesondere Apfelwachse werden bereits von der Kosmetikindustrie in vielfältigen Anwendungsbereichen und insbesondere in Haut- und Haarpflegeprodukten eingesetzt und schützen in Shampoos, Spülungen und Haarkuren die Haut, aber auch das Haar als dünner Film vor Trockenheit und Auslaugung. Beispielhaft sei hier die WO 03/053 394 A2 genannt.

Als Rohstoffquelle für diese Apfelwachse dienen in der Hauptsache Trester, also getrocknete Rückstände aus der Apfelsaft- bzw. Pektinfabrikation. Die in diesem Trester enthaltenen Apfelwachse entstammen den Fruchtschalen von Saftäpfeln. Nachdem diese Fruchtwachse aus dem Trester isoliert und von Chlorophyl- und Pestizidresten gereinigt worden sind, steht in der Regel ein geruchsneutraler Rohstoff zur Verfügung. Dieses Rohmaterial ist ohne weiterreichende Stoffumwandlung zugänglich, d. h. es kann durch rein physikalisch wirkende Methoden wie bspw. Adsorption, Filtration und Destillation gereinigt und gewonnen werden. Der Rückstand aus der Fruchtwachsgewinnung kann, wie der fruchtwachshaltige Trester auch, als Viehfutter eingesetzt werden.

Die bereits angesprochene Isolierung der Fruchtwachse erfolgt bislang mit Hilfe von überkritischem Kohlendioxid, bei dem es sich um ein Lösemittel handelt, das seit Jahrzehnten für die Lebensmittelbehandlung unbedenklich eingesetzt werden kann. Durch die entsprechende Extraktion und/oder Reinigung mit verdichtetem Kohlendioxid erhält man Apfelwachse in Ausbeuten von ca. 2 % in kosmetik-tauglicher Qualität.

Wie bereits angedeutet, stellen natürliche Wachse keine einheitliche Substanzklasse dar, sondern ein Gemisch verschiedener Stoffklassen. Diese Naturextrakte enthalten als Hauptkomponenten Wachsester, Kohlenwasserstoffe, Fettsäuren und andere Verbindungen, wie z. B. Triglyceride, Xanthophyille und Terpene.

Aufgrund der heterogenen Zusammensetzung der bislang für die Fruchtwachsgewinnung eingesetzten Naturextrakte und der bekannten unbedenklichen Eigenschaften von Kohlendioxid war der Einsatz von überkritischem CO₂ bislang das Extraktionsmittel der Wahl. Dabei hat man allerdings die geringe Selektivität billigend in Kauf genommen. Auch verlangt die CO₂-Extraktion hohe Drücke und einen hohen CO₂-Durchsatz, was derartige Verfahren teuer macht. Da die Kosmetikindustrie zunehmend unabhängig vom jeweiligen Anwendungsgebiet reinere Produkte mit definiertem Komponentenspektrum fordert, ist die Extraktion v. a. auch unter wirtschaftlichen Gesichtspunkten mit überkritischem Kohlendioxid nicht mehr ausreichend.

Aufgrund dieses Nachteils des Standes der Technik hat sich für die vorliegende Erfindung die Aufgabe gestellt, ein neues Verfahren zur Gewinnung von Fruchtwachsen aus pflanzlichen Bestandteilen durch Extraktion mit Hilfe verdichteter Gase bereitzustellen, welches den weiter gesteigerten Anforderungen durch die verarbeitende Industrie und durch den Verbraucher gerecht wird. Das neue Verfahren soll auf möglichst einfache Weise kommerziell durchführbar sein und Fruchtwachse in verbesserter Qualität zur Verfügung stellen.

Gelöst wurde diese Aufgabe mit einem Verfahren, das mit Hilfe von verdichtetem Propan und/oder Butan bei Drücken von < 50MPa und Temperaturen von ≤ 70°C durchgeführt wird.

In der Praxis hat sich herausgestellt, dass mit diesem neuen Verfahren gemäß Erfindung nicht nur die Aufgabenstellung erfüllt werden konnte, sondern dass damit überraschenderweise Fruchtwachse in z. T. deutlich gesteigerten Ausbeuten gewonnen werden können. Diese Ausbeuten gehen über die Ausbeuten hinaus, wie sie mit Hilfe von überkritischem Kohlendioxid gemäß Stand der Technik erhältlich sind.

Außerdem sind die mit dem erfindungsgemäßen Verfahren erhältlichen Fruchtwachse von einer Qualität, die die Fruchtwachse neuen Anwendungsgebieten zugänglich machen.

Bezüglich des Ausgangsmaterials hat es sich als günstig erwiesen, Rückstände aus der Obstverarbeitung für das erfindungsgemäße Verfahren einzusetzen, wobei insbesondere Fruchtschalen, z.B. aus der Saftgewinnung und hier besonders bevorzugt Trester und vor allem Nasstrester geeignet sind. Besonders bevorzugt werden die Obstkeme sowie ggf. Rindenstücke, Blätter, Stengel und verholztes Gewebe vor der Extraktion abgetrennt.

Trester, wie z. B. Apfeltrester, bestehen in der Regel aus 10 bis 15 Gew.-% Pektin, aus 20 bis 30 Gew.-% Zuckerstoffen (Fruktose, Glukose, Saccharose) und zu 55 bis 70 Gew.-% aus Nicht-Pektinstoffen, wie z. B. Schalen, Kerne usw. Für die eigentliche Extraktion empfiehlt sich das Trocknen der Nasstrester, wodurch das eigentliche Extraktionsverfahren deutlich wirtschaftlicher durchgeführt werden kann. Vorzugsweise beträgt der Wassergehalt des Extraktionsguts ≤ 15 Gew.-%, besonders bevorzugt 1 bis 10 Gew.-%.

Als Extraktionsmittel verwendet die vorliegende Erfindung Propan und Butan sowie Mischungen daraus. Dabei können im Bedarfsfall, dem jeweils als Extraktionsmittel eingesetzten verdichteten Kohlenwasserstoff (-Gemisch) auch Schleppmittel wie Dimethylether und Alkohole zugesetzt werden, wobei diese dann vorzugsweise in Anteilen von 0,5 bis 50 Gew.-% zur Anwendung gelangen.

Hinsichtlich der Extraktionsparameter verwendet die vorliegende Erfindung Drücke von < 50 MPa und Temperaturen von ≤ 70 °C, wobei ein Druckbereich zwischen 0,5 und 10 MPa und Temperaturen zwischen 20 und 35 °C als besonders bevorzugt angesehen werden.

In Abhängigkeit vom jeweiligen Ausgangsmaterial (bspw. dessen Feuchtigkeitsgehalt) sollte das Verfahren gemäß vorliegender Erfindung mit einem Extraktionsmitteldurchsatz von 4 bis 20 kg/kg Ausgangsmaterial durchgeführt werden, wobei ein Durchsatz als bevorzugt angesehen wird, der zwischen 5 und 10 kg/kg Ausgangsmaterial liegt.

Zur Abscheidung des Extraktes wird üblicherweise eine Druckabsenkung auf 0.5 MPa bis 1 MPa bar bei Temperaturen zwischen 40 und 48 °C durchgeführt.

Zwar kann das vorgeschlagene Verfahren auch kontinuierlich durchgeführt werden; aus praktischen Gründen sieht die Erfindung allerdings vor, das Verfahren batchweise durchzuführen, da so in leicht nachvollziehbarer Weise die Verfahrensparameter auf das jeweilige Ausgangsmaterial, dessen Beschaffenheit und Zusammensetzung abgestimmt werden können.

Neben dem eigentlichen Verfahren selbst beansprucht die vorliegende Erfindung auch die Verwendung eines mit dem vorgeschlagenen Verfahren extrahierten Fruchtwachses in kosmetischen oder pharmazeutischen Zubereitungen und insbesondere zur Pflege und Behandlung der Haut und deren Anhangsgebilden, worunter vor allen Dingen Haare und Fuß- sowie Fingernägel zu verstehen sind. Unter dem Begriff "Haut" sind im Rahmen der vorliegenden Erfindung natürlich zunächst die Haut selbst, aber auch Schleimhäute sowie die Hautanhangsgebilde zu verstehen, sofern diese lebende Zellen umfassen. Hier sind insbesondere Haarfollikel, Haarwurzel, Haarzwiebel sowie das ventrale Epithel des Nagelbetts, Talgdrüsen und Schweißdrüsen zu verstehen. Die erfindungsgemäße Verwendung umfasst die Pflege sowie die therapeutische und nicht-therapeutische Behandlung.

Mit dem neuen Verfahren zur Extraktion von Fruchtwachsen mit Hilfe von Propan oder Butan können natürliche Fruchtwachse in verbesserter Qualität und gesteigerten Ausbeuten unter äußerst wirtschaftlichen Bedingungen aus Ausgangsmaterialien erhalten werden, die üblicherweise Abfallstoffe darstellen und nun als sekundäre Rohstoffe ihre Anwendung finden.

Das nachfolgende Beispiel belegt die Vorteile des beanspruchten Verfahrens.

### Beispiel

Bei der Herstellung von Apfelsaft aus Saftäpfeln fällt Apfeltrester an, in dem sich auch die Reste der Apfelwachs-haltigen Fruchtschalen befinden.

Nach dem Trocknen des Apfeltresters auf Feuchtigkeitsgehalte von < 15 Gew.-% wurden die Bestandteile wie Kerne, Stängel und Blätter entfernt. Der verbliebene Rest wurde in einem Autoklaven bei 30 bar und 35 °C mit flüssigem Propan extrahiert, wobei der Extraktionsmitteldurchsatz durchschnittlich 7,3 kg/kg Ausgangsmaterial betrug. Der so erhaltene Extrakt wurde nach erfolgter Druckabsenkung bei 8 bar und 46 °C abgeschieden.

Auf diese Weise erhielt man einen gelben, geruchsneutralen Apfelwachsextrakt mit einer Ausbeute von etwa 2,5 %.

## Patentansprüche

1. Verfahren zur Gewinnung von Fruchtwachsen aus pflanzlichen Bestandteilen durch Extraktion mit Hilfe verdichteter Gase, **dadurch gekennzeichnet, dass** es mit Hilfe von verdichtetem Propan und/oder Butan als Extraktionsmittel bei Drücken von < 50 MPa und Temperaturen von ≤ 70°C durchgeführt wird.

2. Verfahren nach Anspruch 1, **dadurch gekennzeichnet, dass** als Ausgangsmaterial Rückstände aus der Obstverarbeitung, insbesondere Fruchtschalen aus der Saftgewinnung, bevorzugt Trester und insbesondere Nasstrester, eingesetzt werden.

3. Verfahren nach einem der Ansprüche 1 oder 2, **dadurch gekennzeichnet, dass** der Druck auf 0,5 bis 10 MPa und die Temperatur auf 20 bis 35°C eingestellt werden.

4. Verfahren nach einem der Ansprüche 1 bis 3, **dadurch gekennzeichnet, dass** dem verdichteten Kohlenwasserstoff-Gemisch Schleppmittel wie Dimethylether und Alkohole, vorzugsweise in Anteilen von 0,5 bis 50 Gew.-% zugesetzt werden.

5. Verfahren nach einem der Ansprüche 1 bis 4, **dadurch gekennzeichnet, dass** die Extraktion mit einem Extraktionsmitteldurchsatz von 4 bis 20 kg/kg Ausgangsmaterial und bevorzugt 5 bis 10 kg/kg durchgeführt wird.

6. Verfahren nach einem der Ansprüche 1 bis 5, **dadurch gekennzeichnet, dass** es batchweise durchgeführt wird.

7. Verwendung eines nach dem Verfahren gemäß Ansprüchen 1-6 extrahierten Fruchtwachses in kosmetischen Zubereitungen, insbesondere zur Pflege der Haut und deren Anhangsgebilden.

8. Extrahiertes Fruchtwachs erhältlich nach einem Verfahren gemäß einem der Ansprüche 1-6 zur Anwendung in einer pharmazeutischen Zubereitung, vorzugweise zur Behandlung der Haut.

## Claims

1. Method for obtaining fruit waxes from plant constituents by extraction with the help of compressed gases, **characterized in that** it is carried out with the help of compressed propane and/or butane as extraction agent at pressures of < 50 MPa and temperatures of ≤ 70°C.

2. Method according to Claim 1, **characterized in that** residues from the processing of fruit, in particular fruit peels from the production of juice, preferably pomaces and in particular wet pomaces, are used as starting material.

3. Method according to either Claim 1 or 2, **characterized in that** the pressure is adjusted to 0.5 to 10 MPa and the temperature is adjusted to 20 to 35°C.

4. Method according to any of Claims 1 to 3, **characterized in that** entrainers such as dimethyl ether and alcohols, preferably in amounts of from 0.5 to 50% by weight, are added to the compressed hydrocarbon mixture.

5. Method according to any of Claims 1 to 4, **characterized in that** the extraction is carried out with an extraction agent throughput of from 4 to 20 kg/kg of starting material and preferably 5 to 10 kg/kg.

6. Method according to any of Claims 1 to 5, **characterized in that** it is carried out batchwise.

7. Use of a fruit wax extracted by the method according to Claims 1 to 6 in cosmetic preparations, in particular for the care of the skin and appendages thereof.

8. Extracted fruit wax obtainable by a method according to one of Claims 1 to 6 for use in a pharmaceutical preparation, preferably for the treatment of the skin.

## Revendications

1. Procédé pour l'obtention de cires de fruits à partir de composants végétaux par extraction à l'aide de gaz comprimés, **caractérisé en ce qu'**on l'effectue à l'aide de propane et/ou de butane comprimé(s) en tant qu'agent d'extraction, sous des pressions < 50 MPa et à des températures ≤ 70 °C.

2. Procédé selon la revendication 1, **caractérisé en ce qu'**on utilise comme produit de départ des résidus de la transformation de fruits, en particulier des peaux de fruits provenant de l'extraction de jus, de préférence des marcs et en particulier des marcs humides.

3. Procédé selon la revendication 1 ou 2, **caractérisé en ce qu'**on règle la pression à 0,5-10 MPa et la température à 20-35 °C.

4. Procédé selon l'une quelconque des revendications 1 à 3, **caractérisé en ce qu'**on ajoute au mélange d'hydrocarbures comprimé des agents d'entraînement tels que l'éther diméthylique et des alcools, de préférence en proportions de 0,5 à 50 % en poids.

5. Procédé selon l'une quelconque des revendications 1 à 4, **caractérisé en ce qu'**on effectue l'extraction à un débit d'agent d'extraction de 4 à 20 kg/kg de produit de départ et de préférence de 5 à 10 kg/kg.

6. Procédé selon l'une quelconque des revendications 1 à 5, **caractérisé en ce qu'**on l'effectue en mode discontinu.

7. Utilisation d'une cire de fruit extraite conformément à un procédé selon les revendications 1 à 6, dans des préparations cosmétiques, en particulier destinées au soin de la peau et de ses annexes.

8. Cire de fruit extraite pouvant être obtenue conformément à un procédé selon l'une quelconque des revendications 1 à 6, pour utilisation dans une préparation pharmaceutique, de préférence destinée au traitement de la peau.
